# EUROPEAN PATENT APPLICATION

(11) **EP 1 132 074 A1**
(43) Date of publication of application: **12.09.2001**
(21) Application number: 00940858.4
(22) Date of filing: 28.06.2000
(51) Int. Cl.: A61K 7/00, A61K 7/48

(54) **COSMETICS**

(30) Priority: 14.09.1999 JP 25978199
(71) Applicant: Horiuchi, Isao, Higashi-Yatsushiro-gun, yamanashi 405-0056 (JP)
(72) Inventor: Horiuchi, Isao, Higashi-Yatsushiro-gun, yamanashi 405-0056 (JP)
(74) Representative: Bubb, Antony John Allen
(86) International application number: JP0004223
(87) International publication number: WO0119324

(57) **Abstract**

A cosmetic is provided, which is prepared by mixing papain, bromelain, a protease derived from Agaricus blazei Muril, a protease derived from Grifola frondosa, or a pretease derived from Bacillus natto, and the culture supernatant of lactic acid bacteria such as lactic acid bacteria for fermenting kefir. By using such a cosmetic, it is possible to remove the pigmented cells such as chloasmata, freckles, and lentigines.

## Description

### Technical Field

The present invention relates to cosmetics. More particularly, it relates to a cosmetic capable of removing chloasmata, freckles, lentigines, and the like.

### Background Art

It is known in the art that an ascorbic acid is used internally as the treatment method of pigmentation diseases such as chloasmata and freckles, and that it is also used with a stabilizer mixed therein as a cosmetic (Japanese Patent Publication Nos.Sho 54-974 and 55-43443, and the like). It is also known that hydroquinone is effective for the treatment of chloasmata, freckles, and the like. However, hydroquinone is also unstable to heat, air, and the like. Accordingly, there are publicly known a skin external preparation in which arbutin, which is converted to hydroquinone in the skin, is mixed (Japanese Patent Publication No.Sho 60-16906, and the like), and a whitening cosmetic in which a hydroquinone derivative is mixed with a kojic acid (Japanese Patent Publication No.Sho 32-8100). In addition, there are proposed a cosmetic extracted from an animal organ (Japanese Laid-Open Patent Publication No.Sho 63-8312, and the like), a cosmetic in which a culture solution of Saccharomyces yeast separated from kefir granules serving as a starter of fermented milk kefir is mixed (Japanese Laid-Open Patent Publication No.Hei 7-10734, and the like), etc.

Further, as cosmetics utilizing lactic acid bacteria, there are known a cosmetic containing the cell body of the Lactobacillus genus or its cell wall component for the preventive treatment of skin erythema occurring upon ultraviolet irradiation (Japanese Laid-Open Patent Publication No.Hei 5-17363), and a skin external preparation containing a crude drug extract such as Souttelaria root extract, and a lactic acid bacteria fermented solution for inhibiting coloring of the skin upon ultraviolet irradiation (Japanese Laid-Open Patent Publication No.Hei 5-238925). Further, there is proposed a cosmetic containing the cell body extract of the Lactobacillus lactic acid bacteria separated from kefir granules for inhibiting melanin formation (Japanese Laid-Open Patent Publication No.Hei 5-163134), or the like.

However, many of the prior-art cosmetics do not have a sufficient effect of removing chloasmata and freckles, but inhibit the formation of chloasmata or freckles, thus creating the need for the development of a cosmetic having a sufficient removing effect thereof.

Under such circumstances, it is therefore an object of the present invention to provide a cosmetic excellent in effect of removing chicasmata, freckles, and the like.

### Disclosure of the Invention

A cosmetic in accordance with the present invention characterized in that comprises a protease, and a culture supernatant of lactic acid bacteria.

Further, in accordance with another feature of the present invention, the protease is papain.

In accordance with a still another feature of the present invention, the protease is bromelain.

In accordance with a further feature of the present invention, the protease is the protease derived from Agaricus blazei Muril.

In accordance with a still further feature of the present invention, the protease is the protease derived from Grifola frondosa.

In accordance with an even further feature of the present invention, the protease is the protease derived from Bacillus natto.

In accordance with an even other feature of the present invention, the lactic acid bacteria are the lactic acid bacteria for fermenting kefir.

In accordance with an even still other feature of the present invention, one or more materials selected from the group consisting of water, glycerin, and collodion are further mixed.

With such a constitution, old pigmented skin cells and dead cells are decomposed by utilizing the enzyme actions of the proteases as described above, so that chloasmata, freckles, lentigines, and the like are removed.

### Best Mode for Carrying Out the Invention

Below, a cosmetic of the present invention will be described in more details.

In the present invention, examples of the protease include the ones of plant origin such as papain, bromelain, and ficin, the ones of animal origin such as pepsin, trypsin, chymotrypsin, and rennin, and proteases of fungi origin such as basidiomycetes and microorganisms. Examples of the basidiomycetes include Agaricus blazei Muril, Grifola frondosa, Tricholoma conglobatum, Lentinus edodes, Fuscoporia obliqua, Pheilinus linteus, Sparassis crispa, and Aericium erinaceum. The proteases contained in the fruit bodies and the mycelia of these basidiomycetes can be used. Mycelia are also present in a waste bed after gathering cultivated fruit bodies, and they are also usable. Examples of microorganisms include Bacillus natto and Bacillus aubtilis.

Out of these proteases, papain, bromelain, and the proteases derived from Agaricus blazei Muril, and derived from Grifola frondosa, and the protease derived from Bacillus natto are preferably used. Papain and bromelain are widely used in the food field. Agaricus blazei Muril is used for health food. Further, Grifola frondosa is also used in the uncooked form for food, and in the dried form for health food. Bacillus natto is utilized for producing natto, i.e., Japanese traditional food. Thus, these are preferred because of their excellence in safety. Out of these, papain is a proteolytio enzyme contained in the milk of the fruit of a papaya, and bromelain is a proteolytic enzyme contained in the epidermis or flesh of the fruit of a pineapple. They are commercially available. The commercially available ones can be used in the present invention. These proteases may be used in combination of two or more thereof.

Agaricus blazei Muril is a kind of Agaricus mushrooms which originally came from Piedate in the suburbs of Sao Paulo in Brazil, and has a white campanulate fruit body like the mushroom of the same genus (Agaricus bisuporous). In recent years, it is known that the polysaccharide derived from Agaricus blazei Muril has an ability of activating a macrophage or an interferon in the cell tissue of a mouse or the like, and it also has a large power of preventing virus from invading a cell. Accordingly, it has been proposed that the polysaccharide is applied to the immunotherapy of cancer. Further, it has been proposed that the protein polysaccharide derived from the fruit body, or a nucleic acid thereof is utilized as an antitumor drug. Under such circumstances, it has become widely cultivated.

Grifola frondosa occurs in a giant cluster of a large number of overlapping fan-shaped or spatulate caps at the root of mainly a fagaceous large tree such as Japanese oak, beech, or pasania. It is very fragrant and flavorsome. and hence it is recognized as a sterling edible fungus. In recent years, the dried product thereof is processed as health food, so that it becomes cultivated in many places.

In the present invention, the proteases derived from Agaricus blazei Muril and derived from Grifola frondosa may be generally cultivated or extracted from the wild one. As the proteases of a mushroom, two types of isozymes in the vicinity of an optimum pH 4 and in the vicinity of pH 7 are generally known, and it is favorable that they are extracted with buffers of their respective pHs. For example, it is favorable that, by using a citric acid - sodium citrate buffer in the vicinity of pH 4, and a monosodium phosphate - disodium phosphate buffer in the vicinity of pH 7 in an amount of, for example, 1 to two times the weight of the fruit body and/or mycelium as extracting solvents, the fruit body and/or mycelium is placed together in a mill to crush for extraction. After crushing, the solid content is separated from the crushed material, and the extracted solution is freeze-dried, and then purified to be used as proteases. An Unpurified one can also be-used as a protease. However, the extraction method of the protease is not limited thereto, and a general enzyme extraction method can be adopted.

The present inventor,etc determined the activities of the proteases obtained by the extraction method as described above in accordance with the method for determining the proteases contained in basidiomycetes ("Handbook for Using Enzyme" edited by Michio Ozaki, published by CHIJINSHOKAN Co., Ltd., p.207 (1980)). Specifically, 200 g of a fruit body and 200 ml of each extracting solvent (both, 0.1 M) were placed in a mill, and crushed for 5 minutes two times for extraction. Then, the protease activities of the filtrates obtained from filtration were determined. The measured results indicated that the activity of the protease extracted at pH 4.0 from the fruit body of Agarics blazei Muril was 46.9 unit/ml, while the activity of the protease extracted at pH 7.0 therefrom was 82.4 unit/ml. The activity of the protease derived from the fruit body of Grifola frondosa extracted in the same manner was 505.1 unit/ml with extraction at pH 7.0, and the activity for papain (188-00171, manufactured by Wako Pure Chemical Industries, Ltd.) determined at the same time was 117.6 unit/ml.

Incidentally, in this measuring method, to 5 ml of a substrate (0.5 % casein) held at 30 °C, is added 1 ml of a protease solution (1 mg/ml for a papain powder), and the mixture is allowed to react for 10 minutes. Thus, such an enzyme titer as to form a TCA soluble product in an amount corresponding to 1 *µ*g of tyrosine per minute is taken as one unit. Specifically, it is calculated by multiplying the value of absorbance increased due to the enzyme reaction by 149.

The protease derived from Bacillus natto may be referred to as natto kinase, It is a proteolytic enzyme which has a high thrombolytic action, and is said to be applicable to the treatment and prevention of thrombosis such as cardiac infarction or cerebral infarction. With experiments by the present inventor,etc, when it was applied to the ahloasma (dead cell) on the arm, it exhibited such high lysis effects that the chloasma was reduced in size and also color in several days. The natto kinase one of the strong and safe ones as the components of the cosmetic of the present invention.

With the cosmetic of the present invention, pigmented old skin cells and dead cells are decomposed by the foregoing proteases to remove the chloasmata, freckles, lentigines, and the like, wherein the enzyme actions of the proteases are utilized.

The lactic acid bacteria are classified into a coccus and a bacillus according to the bacteria form. Examples of the coccus include Streptococcus, Leuconostoc, and Pediococcus, and examples of the bacillus include Laotobacillus and Bifidobacterium. It is construed that any lactic acid bacteria of the foregoing genuses are included in the scope of the present invention, and that non-natural type lactic acid bacteria such as recombinant lactic acid bacteria obtained by a genetic recombination method, and a lactic acid bacteria mutant obtained by artificially inducing variation therein are also included in the scope of the invention. Preferred lactic acid bacteria have no pathogenicity against a human and other animals, and they are commercially available lactic acid bacteria for use in the manufacturing of foods such as fermented dairy products, fermented meet products, brew products, fermented soybean milk, and pickled vegetables. Such lactic acid bacteria are commercially available from, for example, Chr. Hansen's Co.

As the examples of the lactic acid bacteria usable in the present invention, mention may be made of, for example, Streptococcus thermophilus, Streptococcus lactis, and Streptococcus lactis subap. diacetilactis as the examples of the Streptococcus genus. Examples of the Leuconostoc genus include Leuconostoc cremoris and Leuconostoc oenos. Examples of the Pediococcus genus include Pediococcus cerevisiae, Pediococcus acidilactici, Pediococcus penntosaceus, Pediococcus halophilus, and Pediococcus urinae-equi.

Further, examples of the Lactobacillus genus include Lactobacillus delbruecki. Lactobacillus leichmannii, Lactobacillus lactis, Lactobacillus bulgaricus, Lactobacillus helveticus, Lactobacillus fermentum, Lactobacillus brevis, and Lactobacillus viridescens. Examples of the Bifidobacterium genus include Bifidobacterium longum, Bifidobacterium bifidum, Bifidobacterium breve, and Bifidobacterium infantis.

Out of these lactic acid bacteria, as the preferred example, mention may be made of the lactic acid bacteria used for the manufacturing of fermented milk kefir. Kefir is fermented milk which is said to have supported the health of the people in the Caucasus region known as a longevity country. The starter thereof is called kefir granules, which contain the lactic acid bacteria of Streptococcus lactis and Lactobacillus bulgaricus described above.

The culture supernatant of the lactic acid bacteria in the present invention is the supernatant liquid of the lactic acid bacteria culture solution as shown above, and it can be obtained by culturing lactic acid bacteria, and removing the cell bodies from the resulting culture solution.

In culturing lactic acid bacteria, there is no particular restriction. Any culturing method can be adopted as long as it is carried out under such conditions that the lactic acid bacteria can be sufficiently grown. However, conditions such as the culture medium, temperature, pH, and culturing time can vary according to the type of the lactic acid bacteria. As for the culturing conditions and the culturing method, there can be used the ones described, for example, in "Bergey's Manual of Determinative Bacteriology" (8th edition, 1974), and in the instruction manual for the commercially available lactic acid bacteria provided from its manufacturer.

As the culture medium component, there can be used any given combination of one or more of carbon sources and nitrogen sources such as milk serum (whey), glucose, lactose (milk sugar), and peptone. Animal milks such as cow milk, goat milk, or human milk can be used in place of whey. The culturing temperature varies according to the type of bacteria, but it is the generally known temperature (about 20 to 45 °C). As for the heat-resistant lactic acid bacteria, they can be cultured at a temperature higher than this. Further, the culturing time is from several hours to 72 hours, and preferably from about 15 to about 50 hours. The amount of the lactic acid bacteria to be inoculated is in the range of from 10 to 100 ml per liter of the culture medium.

Below, one example of the culturing conditions will be shown, but it is not exclusive.

For the lactic acid bacteria of the Streptococcus genus, Leuconostoc genus, and Lactobacillus genus, culturing can be performed in MRS medium (commercially available from Difco Co.) at 30 to 37 °C, and at a pH of 6.8, for 24 to 48 hours. For the Pediococcus lactic acid bacteria, culturing can be performed in MRS medium with 1 to 3 % sodium chloride added therein at 35 to 40 °C, and at a pH of 6.8, for 24 to 48 hours.

For the Bifidobacterium lactic acid bacteria, culturing can be performed in BL medium (commercially available from Wako Pure Chemical Industries, Ltd.), or EG medium (commercially available from Merok Co.) at 30 to 37 °C, and at a pH of 7 to 8, for 24 to 48 hours under anaerobic conditions.

In accordance with the specific example of the present invention, to a culture medium containing a milk serum and a milk sugar(for example, 10 % and 5 %, respectively), lactic acid bacteria are inoculated, followed by still standing, or shaking culture at 35 to 37 °C for about 24 hours. After culturing, the cell bodies can be removed from the culture solution by using a separation means such as centrifugation and filtration to recover the culture supernatant.

The culture supernatant of the lactic acid bacteria controls the enzyme action, i.e., the cytolysis action of the protease to stabilize the live cell, thus exerting a kind of a buffer effect. Further, the culture supernatant of the lactic acid bacteria contains therein various nutrients such as vitamins and minerals as the culture medium and fermented products, and resupplies these nutrients to the skin. Consequently, it provides a moisture retention effect and activates the live cell, and further it also exerts an effect of promoting new formation of the skin cell.

In the cosmetic of the present invention, a protease and the culture supernatant of lactic acid bacteria are preferably mixed in amounts of from 0.1 to.5 % by weight, and from 1 to 50 % by weight, respectively, based on the total amount of the cosmetic. If the amount of the protease to be mixed is less than 0.1 % by weight, the enzyme action is insufficient. On the other hand, if it exceeds 5 % by weight, the enzyme action is too strong, whereby the live cell is damaged. Further, if the amount of the culture supernatant of the lactic acid bacteria is less than 1 % by weight, the buffer effect is not enough. On the other hand, it has no particular upper limit. However, if it exceeds 50 % by weight, the effect is saturated, and hence this amount not exceeding 50 % by weight is enough. More preferably, the amount of the protease to be mixed is from 0.2 to 2 % by weight for papain, from 0.1 to 1 % by weight for bromelain, and from 0,1 to 4 % by weight for the proteases derived from Agaricus blazei Muril, derived from Grifola frondosa, and derived from Bacillus natto, and the amount of the culture supernatant of the lactic acid bacteria is from 5 to 25 % by weight.

When the cosmetic of the present invention is formed into a preparation, in order to control the concentration in the case where the protease and the culture supernatant of the lactic acid bacteria are applied to the skin. they can be dissolved in water or a water - alcohol mixed solution to a concentration of 100 % by weight to form a cosmetic in liquid form. Alternatively, they can also be formed in cream form by using a cosmetic base such as vaseline in place of water.

As the bases for a cosmetic, mention may be made of, other than the vaseline, the ones known as conventional cosmetic bases such as liquid paraffin, cetyl alcohol, stearyl alcohol, polyoxyethylene hardened castor oil, polyoxyethylene sorbitan monostearate, cetostearyl alcohol, adsorption purified lanolin, and methyl paraben.

Further, as the medium when applied to the skin, the moisture retention effect can be provided by mixing glycerin therein, and by adding collodion later, the collodion can form a film to provide an effect of fixing the cosmetic of the present invention on a skin surface. Still further, an ascorbic acid known as a whitening agent in the art may be added thereto. These may be mixed in an amount conventionally to be mixed in the cosmetic. In general, glycerin may be mixed in an amount of from 1 to 20 % by weight, and collodion may be mixed in 1 to 2 drops (about 0.2 ml/100 ml) when a film is topically required.

Any one or more of the foregoing water, glycerin, collodion, and ascorbic acid can be mixed with a protease and the culture supernatant of the lactic acid bacteria to form a cosmetic.

Further, when the cosmetic of the present invention is a liquid or an emulsion, methyl paraben, ethyl paraben, or the like can be mixed therein as a preservative, and when it is a cream, ethyl paraben, propyl paraben, or the like can be mixed therein as a preservative. As the preservative, in addition to these materials. there can be used a benzoic acid, a sorbic acid, propylene glycol, or the like. The mixing amounts are from 0.1 to 3 % by weight for parabens, generally not more than 0.5 % by weight for a benzoic acid or a sorbic acid, and from 3 to 40 % by weight for propylene glycol.

The cosmetic of the present invention can take the form of a solution, cream, or the like as described above. If it is applied in an appropriate amount several times per day to the pigmented skin such as skin having chloasmata including senile chloasmata, freckles, lentigines, or the like, the pigmented skin is reduced in color as the days goes on, or the freckles are gradually reduced in size, so that the chloasmata or freckles can be removed in several days to ten and several days. Especially, since the senile spots are dead cells, they are more likely to be decomposed by the enzyme as compared with live cells, and hence they are removed more effectively.

### [Examples]

The present invention will be described more specifically by way of examples below. It is noted that "%" denotes "% by weight" in the examples.

### Reference Example 1 (production of lactic acid bacteria culture supernatant)

To a 5-L Erlenmeyer flask, was charged 4 L of distilled water. A 10 % whey powder (manufactured by Meiji Milk Products Co., Ltd.) and a 5 % milk sugar were added thereto. The resulting mixture was inoculated with 0.1 % kefir-fermenting lactic acid bacteria (commercially available from Chr. Hansen's), followed by stationary culturing at 37 °C for 24 hours. The resulting culture solution had a pH of 4.0. The culture solution was subjected to centrifugation to separate the supernatant, which was then used as a culture supernatant for each cosmetic of the following examples.

### Example 1

To 50 g of the culture supernatant obtained in Reference Example 1, 35 g of glycerin (first class grade reagent) was added with stirring. Then, 0.5 g of papain (164-00172, manufactured by Wako Pure Chemical Industries, Ltd.) was dissolved therein, to which 15 g of distilled water was added with stirring to obtain a liquid cosmetic. The resulting liquid cosmetic was applied to the freckled area of the back of each hand of A to C (specimens in Table) (note: A and B denote adult males, and C denotes an adult female) 3 to 4 times per day. The results thereof are shown in Table 1. The results are shown as the variations in size (unit: mm) of each freckle determined by means of a vernier calipers.

Table 1 indicates that the freckle was gradually reduced in size, and disappeared 7 to 10 days later.

**[Table 1]**

| Specimen | Before application | 3 days later | 7 days later | 10 days later |
|---|---|---|---|---|
| A | 2.2 x 1.8 | 1.2 x 1.0 | 0 | - |
| B | 5.2 x 4.9 | 2.0 x 2.1 | 1.2 x 1.2 | 0 |
| C | 4.8 x 3.5 | 2.1 x 1.4 | 0.2 x 1.0 | 0 |

### Example 2

A liquid cosmetic was obtained in the same manner as in Example 1, except that 0.5 g of bromelain (manufactured by Wako Pure Chemical Industries, Ltd.) was dissolved therein in place of papain. The resulting liquid cosmetic was applied to the freckled area of the back of each hand of D to F (note: D and E denote adult males, and F denotes a female) 3 to 4 times per day. The results thereof are shown in Table 2.

**[Table 2]**

| Specimen | Before application | 3 days later | 7 days later | 10 days later |
|---|---|---|---|---|
| D | 2.5 x 1.9 | 1.2 x 1.2 | 0 | - |
| E | 6.0 x 2.5 | 3.2 x 1.4 | 1.5 x 1.0 | 0 |
| F | 0.8 x 0.6 | 0 | - | - |

### Example 3

A liquid cosmetic was obtained in the same manner as in Example 1, except that 0.25 g of papain and 0.25 g of bromelain were dissolved therein in place of 0.5 g of papain. The resulting liquid cosmetic was applied to the freckled area of the back of each hand of G to I (note: G and H denote adult males, and I denotes a female) 3 to 4 times per day. The results thereof are shown in Table 3.

**[Table 3]**

| Specimen | Before application | 3 days later | 7 days later | 10 days later |
|---|---|---|---|---|
| G | 5.1 x 5.5 | 4.2 x 2.3 | 1.2 x 1.1 | 0 |
| H | 2.3 x 1.7 | 1.1 x 1.1 | 0 | - |
| I | 1.8 x 1.8 | 0.5 x 0.5 | 0 | - |

### Example 4

To 30 g of the culture supernatant obtained in Reference Example 1, 20 g of glycerin was added with stirring. Then, 1.0 g of papain was dissolved therein, to which 50 g of a hand cream (SILK hand cream by Kanebo Ltd.) with well stirring, resulting in a cream cosmetic. The resulting cream cosmetic was applied to the freckled area of the back of each hand of J to L (J and K denote adult males, and L denotes a female) 3 to 4 times per day. The results thereof are shown in Table 4.

**[Table 4]**

| Specimen Specimen | Before application | 3 days later | 7 days later | 10 days later |
|---|---|---|---|---|
| J | 3.6 x 2.2 | 1.4 x 1.2 | 0 | - |
| K | 2.8 x 4.0 | 2.0 x 2.4 | 0 | - |
| L | 1.8 x 1.6 | 0 | - | - |

### Example 5

A cream cosmetic was obtained in the same manner as in Example 4, except that 1.0 g of bromelain (manufactured by Wako Pure Chemical Industries, Ltd.) was dissolved therein in place of 1.0 g of papain. The resulting cream cosmetic was applied to the freckled area of the back of each hand of M to O (note: M and N denote adult males, and O denotes a female) 3 to 4 times per day. The results thereof are shown in Table 5.

**[Table 5]**

| Specimen | Before application | 3 days later | 7 days later | 10 days later |
|---|---|---|---|---|
| M | 4.2 x 4.8 | 2.4 X 2.2 | 1.0 x 1.0 | 0 |
| N | 2.8 x 2.1 | 1.2 x 1.0 | 1.0 x 0.5 | 0 |
| O | 4.1 x 4.0 | 2.8 x 2.4 | 1.2 x 1.1 | 0 |

### Example 6

A cream cosmetic was obtained in the same manner as in Example 4, except that 0.5 g of papain and 0.5 g of bromelain were dissolved therein in place of 1.0 g of papain. The resulting cream cosmetic was applied to the freckled area of the back of each hand of P to R (note: P and Q denote adult males, and R denotes a female) 3 to 4 times per day. The results thereof are shown in Table 6.

**[Table 6]**

| Specimen | Before application | 3 days later | 7 days later | 10 days later |
|---|---|---|---|---|
| P | 5.2 x 4.6 | 3.5 x 3.2 | 1.5 x 1.5 | 0 |
| Q | 3.2 x 3.2 | 2.1 x 2.0 | 1.0 x 1.0 | 0 |
| R | 3.2 x 2.8 | 3.0 x 0.8 | 0 | - |

### Example 7

The liquid cosmetic (containing 0.5 g of papain) used in Example 1 was applied to the chloasma area of the back of each hand of 1 to 3 (1 and 2 denote males in their sixties, and 3 denotes a female in her fifties) once per day. The results thereof are shown as variations in ohromatioity (visually observed) in Table 7.

**[Table 7]**

| Specimen | Before application | 3 days later | 7 days later | 10 days later |
|---|---|---|---|---|
| 1 | 4.3 x 4.0 | +++ | + | - |
| 2 | 4.0 x 3.2 | ++ | + | - |
| 3 | 2.5 x 2.8 | + | - | - |
| Note: in the table, +++ represents no change in the depth of the color, ++ denotes a little reduction in color, + denotes a little colored, and - denotes no difference from the color of the skin. | | | | |

### Example 8

The liquid cosmetic (containing 0.5 g of bromelain) used in Example 2 was applied to the chloasma area of the back of each hand of 4 to 6 (4 and 5 denote males in their sixties, and 6 denotes a female in her fifties) once per day. The results thereof are shown as variations in chromaticity in Table 8.

**[Table 8]**

| Specimen Specimen | Before application | 3 days later | 7 days later | 10 days later |
|---|---|---|---|---|
| 4 | 4.0 x 3.2 | ++ | + | + |
| 5 | 6.5 x 5.4 | ++ | + | - |
| 6 | 2.8 x 2.6 | + | - | - |

### Example 9

The liquid cosmetic (containing 0.25 g of papain and 0.25 g of bromelain) used in Example 3 was applied to the chloasma area of the back of each hand of 7 to 9 (7 and 8 denote males in their sixties, and 9 denotes a female in her fifties) once per day. The results thereof are shown as variations in chromaticity in Table 9.

**[Table 9]**

| Specimen | Before application | 3 days later | 7 days later | 10 days later |
|---|---|---|---|---|
| 7 | 4.8 x 4.3 | ++ | + | - |
| 8 | 8.4 x 6.8 | +++ | ++ | + |
| 9 | 2.0 x 1.9 | - | - | - |

### Example 10

The cream cosmetic (containing 1.0 g of papain) used in Example 4 was applied to the chloasma area of the back of each hand of 10 to 12 (10 and il denote males in their sixties, and 12 denotes a female in her fifties) once per day. The results thereof are shown as variations in chromaticity in Table 10.

**[Table 10]**

| Specimen | Before application | 3 days later | 7 days later | 10 days later |
|---|---|---|---|---|
| 10 | 4.8 x 4.6 | ++ | ++ | - |
| 11 | 3.4 x 2.8 | ++ | + | - |
| 12 | 6.4 x 3.9 | +++ | ++ | + |

### Example 11

The cream cosmetic (containing 1.0 g of bromelain) used in Example 5 was applied to the chloasma area of the back of each hand of 13 to 15 (13 and 14 denote males in their sixties, and 15 denotes a female in her fifties) once per day. The results thereof are shown as variations in chromaticity in Table 11.

**[Table 11]**

| Specimen | Before application | 3 days later | 7 days later | 10 days later |
|---|---|---|---|---|
| 13 | 4.7 x 4.0 | +++ | ++ | - |
| 14 | 3.6 x 3.3 | ++ | + | + |
| 15 | 6.4 x 5.5 | +++ | ++ | + + |

### Example 12

The cream cosmetic (containing 0.5 g of papain and 0.5 g of bromelain) used in Example 6 was applied to the chloasma area of the back of each hand of 16 to 18 (16 and 17 denote males in their sixties, and 18 denotes a female in her fifties) once per day. The results thereof are shown as variations in chromatioity in Table 12.

**[Table 12]**

| Specimen Specimen | Before application | 3 days later | 7 days later | 10 days later |
|---|---|---|---|---|
| 16 | 4.7 x 4.0 | +++ | ++ | - |
| 17 | 3.6 x 3.3 | ++ | + | + |
| 18 | 6.4 x 5.5 | +++ | ++ | + |

### Example 13

Ten gram of the culture supernatant obtained in Reference Example 1, 10 g of glycerin, 1.0 g of papain, and 79 g of vaseline were well mixed to prepare a cream cosmetic, which was then respectively applied to the freckled areas (S to U) of the backs of hands to determine their respective changes. The results are shown in Table 13. For comparison, a test was also carried out in the same manner for a cosmetic not containing the culture supernatant and papain (V).

**[Table 13]**

| Specimen | Before application | 3 days later | 7 days later | 10 days later |
|---|---|---|---|---|
| S | 3.4 x 3.2 | 2.2 x 2.8 | 2.2 x 2.2 | 1.2 x 1.2 |
| T | 4.0 x 3.8 | 3.6 x 3.0 | 1.8 x 2.8 | 0 |
| U | 3.1 x 2.1 | 1.8 x 1.8 | 1.2 x 1.0 | 0 |
| V | 2.9 x 2.9 | 2.9 x 2.9 | 2.9 x 2.9 | 2.9 x 2.9 |

As apparent from the results of Table 13, no change was observed in the freckle in the blank test (V), while the freckles were reduced with time for the cosmetics of the present invention, thus showing the effects of the cosmetics of the present invention.

### Example 14

A liquid cosmetic was prepared in accordance with the following formulation (weight ratio).

| | |
|---|---|
| Culture supernatant obtained in Reference Example 1 | 10.0 |
| Papain | 1.0 |
| Squalane | 5.0 |
| Stearic acid | 2.5 |
| Cetanol | 2.5 |
| Self-emulsifiable glycerin monostearate | 1.5 |
| Polyoxyethylene cetyl ether | 1.5 |
| Paraben | Proper amount |
| Concentrated glycerin | 3.5 |
| Propylene glycol | 3.5 |
| Total amount is made 100 with purified water Perfume | Proper amount |

### Reference Example 2 (production of protease derived from the fruit body of Agaricus blazei Muril)

The fruit body of raw Agaricus blazei Muril after culturing was washed with water. Then, the proteases were extracted by using two types of buffers, i.e., pH 4.0 (0.1 M citric acid - sodium citrate buffer, for extracting an acidic protease,) and pH 7.0 (0.1 M phosphoric acid - sodium phosphorate buffer, for extracting a metal protease). Specifically, 200 ml of each buffer, and 200 g of the fruit body were placed in a mill (manufactured by Nippon Seiki Co., Ltd., 2.5 L, 180 mmφ), and crushed for 5 minutes two times. Thereafter, the crushed solution was filtered through a nylon mesh (NYLON No. 300, manufactured by Earth Co., Ltd.) to remove the solid content. The supernatant liquid was recovered to be freeze-dried, resulting in a powder. The resulting powder was taken as the protease derived from the fruit body of Agaricus blazei Muril. This protease was stable in a cool, dark place (not more than 5 °C).

### Example 15

A liquid cosmetic was prepared in precisely the same manner as in Example 14, except that 1.0 part of the dried powder of the pH 7.0 extracted solution out of the proteases derived from the fruit body of Agaricus blazei Muril produced in Reference Example 2 was mixed therein in place of 1.0 part of papain. The resulting liquid cosmetic was applied to the ohloasma area of the back of each hand of 19 to 22 (19 and 20 denote males in their sixties, and 21 and 22 denote females in their fifties) 2 to 3 times per day. The results thereof are shown as variations in chromaticity (visually observed) in Table 14.

**[Table 14]**

| Specimen | Before application | 3 days later | 7 days later | 10 days later |
|---|---|---|---|---|
| 19 | +++ | ++ | - | - |
| 20 | +++ | + | - | - |
| 21 | +++ | ++ | - | - |
| 22 | +++ | ++ | - | - |

### Example 16

A liquid cosmetic was prepared in the same manner, except that the powder (protease derived from Grifola frondosa) which had been extracted from the fruit body of Grifola frondosa with a pH 7.0 buffer, and dried in the same manner as in Reference Example 2 was mixed in place of papain in Example 14. The resulting liquid cosmetic was applied to the chloasma area of the back of each hand of 23 to 26 (23 and 24 denote males in their sixties, and 25 and 26 denote females in their fifties) 2 to 3 times per day. Then, the variations in size (the average value of the longitudinal length and the transverse length) of each chloasma were determined by means of a vernier calipers. The measured results are shown in Table 15.

**[Table 15]**

| Specimen | Before application | 3 days later | 7 days later | 10 days later |
|---|---|---|---|---|
| 23 | 0.8 | 0.2 | 0.2 | 0 |
| 24 | 10.5 | 5 | 3 | 3 |
| 25 | 4 | 2 | 0 | - |
| 26 | 22 | 18 | 15 | 15 |

### Industrial Applicability

As described above, the cosmetic in accordance with the present invention is capable of effectively removing chloasmata, freckles, and the like. Further, it is possible to make the cosmetic excellent in moisture retention effect by mixing glycerin therein, or excellent in fixing effect by mixing collodion therein.

## Claims

1. A cosmetic comprising a protease, and a culture supernatant of lactic acid bacteria.

2. The cosmetic according to claim 1, wherein said protease is papain.

3. The cosmetic according to claim 1, wherein said protease is bromelain.

4. The cosmetic according to claim 1, wherein said protease is a protease derived from Agariqus blazei Muril.

5. The cosmetic according to claim 1, wherein said protease is a protease derived from Grifola frondosa.

6. The cosmetic according to claim 1, wherein said protease is a protease derived from Bacillus natto.

7. The cosmetic according to claim 1, wherein said lactic acid bacteria are lactic acid bacteria for fermenting kefir.

8. The cosmetio according to claim 1, wherein one or more materials selected from the group consisting of water, glycerin, and collodion are further mixed.
